# EUROPEAN PATENT SPECIFICATION

(11) **EP 3 614 939 B1**
(45) Date of publication and mention of the grant of the patent: **05.08.2026**
(21) Application number: 18790729.0
(22) Date of filing: 30.04.2018
(51) Int. Cl.: A61B 18/00

(54) **SYSTEM FOR INTERSTITIAL PHOTODYNAMIC THERAPY**
SYSTEM ZUR INTERSTITIELLEN PHOTODYNAMISCHEN THERAPIE
SYSTÈME DE THÉRAPIE PHOTODYNAMIQUE INTERSTITIELLE

(30) Priority: 29.04.2017 US 201762492171 P
(43) Date of publication of application: 04.03.2020
(73) Proprietor: Health Research, Inc., Buffalo, NY 14263 (US)
(72) Inventor: SHAFIRSTEIN, Gal, Amherst, NY 14228 (US); BELLNIER, David, Buffalo, NY 14214 (US)
(74) Representative: Kramer, Dani
(86) International application number: PCT/US2018/030314
(87) International publication number: WO 2018/201156

(56) References cited:
- EP-A1- 2 799 111
- WO-A1-2017/004482
- WO-A1-2017/015674
- US-A- 5 483 958
- US-A- 5 496 308
- US-A- 5 496 308
- US-A1- 2006 052 661
- US-A1- 2006 052 661
- US-A1- 2009 076 489
- US-A1- 2009 076 489
- US-A1- 2015 230 864
- US-A1- 2017 014 186

## Description

### Field of the Disclosure

The present disclosure relates to photodynamic therapy.

### Background of the Disclosure

Photodynamic therapy (PDT), in particular interstitial photodynamic therapy (I-PDT), offers promising outcomes for patients with refractory locally advanced cancer. The use of I-PDT with porfimer sodium (Photofrin^{®}) is approved for palliation in patients with esophageal cancer or lung cancer with airway obstruction, who are non-candidates for surgery or radiation therapy. In addition, I-PDT with porfimer sodium has been used, in compassionate care settings, to treat patients with head and neck squamous cell carcinoma. A principal clinical goal has been to shorten treatment times by administering the therapeutic light at high dose rates (*i.e.,* 400 mW/cm) that are clinically approved by the FDA for I-PDT with porfimer sodium.
However, the cure rate for I-PDT with porfimer sodium is limited.

PDT has also been viewed as beneficial when considering the relatively minor nature of adverse effects. Additionally, it has been noted that PDT provides excellent cosmetic outcomes. To date, it has been believed that during I-PDT the changes in tissue temperature do not affect the response. Physicians and researchers assumed that PDT is associated with minimal heating. The clinically approved, and used, light dose rate (400 mW/cm) for PDT or I-PDT with porfimer sodium was chosen arbitrarily, about 25 years ago. There was no systemic study to evaluate potential tissue heating during I-PDT. Several retrospective clinical studies suggest that PDT and I-PDT will result in retention of functional anatomy and other benefits. While offering improved palliative outcomes for patients with such advanced diseases, there remains a need for further improvement.

US5496308A describes a medical delivery system capable of emitting radiation with wavelengths between 190 nm and 16 um in one or more essentially directed, predetermined patterns.

US20060052661A1 describes a control system for minimally invasive surgery, and more particularly, for laser surgery using infrared laser. A feedback mechanism is provided to obtain thermographic information from the targeted site and a processor uses this thermographic information to monitor and control input parameters, including air flow, suction, and laser beam parameters. Furthermore, an infrared imaging fiber bundle is used in combination with an infrared camera to provide the thermographic information to the processor. Specifically, the system and methods provided can be used to more effectively present very specific wavelengths of laser treatment, with capability of monitoring its effects and altering parameters at the time of treatment. Furthermore, means for thermographic analysis of the targeted area, wherein such analysis provides a guideline for the monitoring and altering of the controllable parameters is provided.

EP2799111A1 describes an apparatus for obtaining an anti-tumour immunologic response by thermotherapy of a treatment lesion covering at least a portion of a tumour is disclosed. The apparatus comprises a heating probe comprising an optical fiber and a cooling catheter. The optical fiber is inserted in the cooling catheter. Further the heating probe has a light emitting area, and the heating probe is interstitially insertable into the tumour of the treatment lesion. The heat probe is internally cooled by a fluid circulating in said catheter. The apparatus further comprises a first thermal sensor member having at least one sensor area. The first thermal sensor member is positionable at a distance adjacent to the heating probe. The apparatus also comprises a control unit for controlling a power output of said light source based on a measured first temperature

WO2017004482A1 describes methods, apparatus, and kits for applying thermal energy to tissue in a region of interest. Certain embodiments include registration of fiducial markers with an image guidance system and temperature monitoring via magnetic resonance imaging thermograpy.

US20170014186A1 describes a device and a method for monitoring and optimizing photothermal therapy, using a high-power continuous-wave laser beam and a pulsed laser beam, both transmitted through a single soft, multi-mode optical fiber with a diffuse active tip, to interstitially irradiate the target tissue at the same time. The continuous-wave laser light induces photothermal effect and increases tissue temperature and the pulsed laser light produces a photoacoustic signal. The photoacoustic signal intensity is used to monitor the temperature changes in the target tissue and to guide the irradiation of the high-power laser to optimize the photothermal effect by adjusting the light intensity and irradiation time.

WO2017015674A1 describes a system and method for interstitial photodynamic light therapy (I-PDT) of a tissue. A plurality of light-transmitting catheters (LTCs) are provided and placed in the tissue according to a pre-determined treatment plan, wherein an LTC includes a first treatment fiber disposed therethrough, and an LTC includes a dosimetry fiber disposed therethrough. A dose light is provided to the tissue by way of the first treatment fiber according to the pre-determined treatment plan. Light received at the dosimetry fiber is measured using a spectrometer in operable communication with the dosimetry fiber. One or more properties of a photosensitizer in the tissue are determined. The treatment plan is modified based on the properties of the photosensitizer, and an updated dose light is provided to the tissue by way of the first treatment fiber according to the modified treatment plan.

US5483958A describes a solid-state fluorescent dosimeter for monitoring therapy irradiation dosage during a photodynamic therapy procedure. The solid-state fluorescent dosimeter includes an optical fiber having a distal end and a proximal end, and a solid-state fluorescent tip attached to the proximal end of the optical fiber. The solid-state fluorescent tip includes a fluorescent material which emits fluorescence when exposed to non-ionizing radiation in the visible or near infrared range. The solid-state fluorescent tip has a sufficient length so as ensure isotropic response characteristics to the non-ionizing radiation regardless of the orientation or alignment of the solid-state fluorescent tip relative to the irradiation source.

### Brief Summary of the Disclosure

The invention relates to a system as defined in the appended claims. Embodiments, examples or aspects in the following disclosure, in particular methods, which do not fall under the scope of the claims are presented for illustration purposes only and do not form part of the invention.

In a first aspect, the present disclosure provides a method for treating a tissue. A photosensitizer is administered to the tissue. One or more optical fibers are placed in the tissue. For example, a portion (such as, for example, an end portion) of the one or more optical fibers are inserted in the tissue. The optical fibers may be spaced apart from one another such that a light dose may be applied to the tissue. The method includes applying a treatment light to the tissue by way of the one or more optical fibers. A temperature of the tissue is measured during application of the treatment light. The fluence rate (mW/cm²) of the treatment light is modified based on the temperature of the tissue. For example, by adjusting the light dose rate one may govern the intratumoral fluence rate within the tissue (e.g., intratumoral fluence rate). For example, the fluence rate may be modified to be lower if the temperature of the tissue is higher than a predetermined threshold. In some embodiments, the fluence rate is modified to maintain a tissue temperature between 50°C and 65°C. In some embodiments, the fluence rate is modified to maintain a tissue temperature of substantially 60°C. In some embodiments, the fluence rate is modified to maintain a tissue temperature between 60°C and 90°C.

In some embodiments, one or more dosimetry fibers may be placed in the tissue and configured to measure light dose (J/cm²). The optical fiber(s) and/or the dosimetry fiber(s) may be disposed within one or more light-transmitting catheters (LTCs) placed in the tissue. For example, each optical fiber may be disposed in a corresponding LTC. In another example, each of a plurality of LTCs may contain an optical fiber and a dosimetry fiber.

In another aspect, the present disclosure may be embodied as a system for treating a tissue. The system includes a light source and an optical fiber operably coupled to the light source. The optical fiber is configured to deliver a light dose of treatment light to the tissue. A temperature sensor is configured to measure a temperature of the tissue. The temperature sensor may be any suitable sensor such as, for example, a thermistor, a thermal imaging sensor, a fiber optic, a magnetic resonance thermometer (providing volumetric temperature data), or the like. In some embodiments, the temperature sensor is configured to measure temperature at a plurality of locations throughout the volume of the tissue. In some embodiments, the temperature sensor comprises a plurality of temperature sensitive catheters.

A controller, such as, for example, a programmable microprocessor, is in communication with the temperature sensor and configured to modify a fluence rate of the treatment light based on a measured temperature. In some embodiments, the controller is configured to modify the fluence rate of the treatment light to maintain a tissue temperature between 50°C and 65°C. In some embodiments, the controller is configured to modify the fluence rate of the treatment light to maintain a tissue temperature of substantially 60°C. According to the invention, the controller is configured to modify the fluence rate of the treatment light to maintain a tissue temperature between 60°C and 90°C.

The system may further include a dosimetry fiber for measuring the light dose. A spectrometer may be operably coupled to the dosimetry fiber. The system may further include an LTC, and the optical fiber and/or the dosimetry fiber may be disposed in the LTC.

In some embodiments, a second optical fiber is operably coupled to the light source and configured to deliver a second light dose of treatment light to the tissue. The controller may be configured to modify a fluence rate of the second light dose based on the measured temperature. The fluence rate of the second light dose may be modified based on a temperature at a second location of the tissue.

### Description of the Drawings

For a fuller understanding of the nature and objects of the disclosure, reference should be made to the following detailed description taken in conjunction with the accompanying drawings, in which:
Figure 1 depicts a system according to an embodiment of the present disclosure;
Figure 2 is a chart showing a method according to another embodiment of the present disclosure;
Figure 3 shows a mouse being fitted with two optical fibers, each disposed within a light-transmitting catheter, for use in interstitial photodynamic therapy (I-PDT);
Figure 4 is a chart showing intratumoral heating results for a light dose of 150 mW/cm, 100 J/cm with and without photosensitizer;
Figure 5 is a chart showing intratumoral heating results for a light dose of 350 mW/cm, 100 J/cm with and without photosensitizer;
Figure 6 is a chart showing tumor size over time in populations of mice, where a control population was untreated, a second population was treated with a light dose of 150 mW/cm, 100 J/cm (control vs. light *p* = < 0.0001), and a third population was treated with I-PDT (control vs. I-PDT *p = <* 0.0001; light vs. I-PDT *p* < 0.05);
Figure 7 is a chart showing tumor size over time in populations of mice, where a control population was untreated, a second population was treated with a light dose of 350 mW/cm, 100 J/cm (control vs. light *p* = < 0.0001), and a third population was treated with I-PDT (control vs. I-PDT *p* = < 0.0001; light vs. I-PDT *p* = 0.339); and
Figure 8 is a chart showing tumor size over time in populations of mice, where a control population was untreated, a second population was treated with a light dose of 100 mW/cm, 540 J/cm (control vs. light *p* = < 0.0001), and a third population was treated with I-PDT concurrent with the same light dose as the second population (control vs. light *p* = < 0.0001; light vs. I-PDT/light *p* = 0.164).

### Detailed Description of the Disclosure

As mentioned above, a perceived benefit of photodynamic therapy has been the lack of significant changes in tissue temperature. However, the present disclosure advantageously utilizes increased temperatures induced in the tissue using the I-PDT techniques disclosed herein to enhance efficacy as compared to I-PDT without an increased temperature.

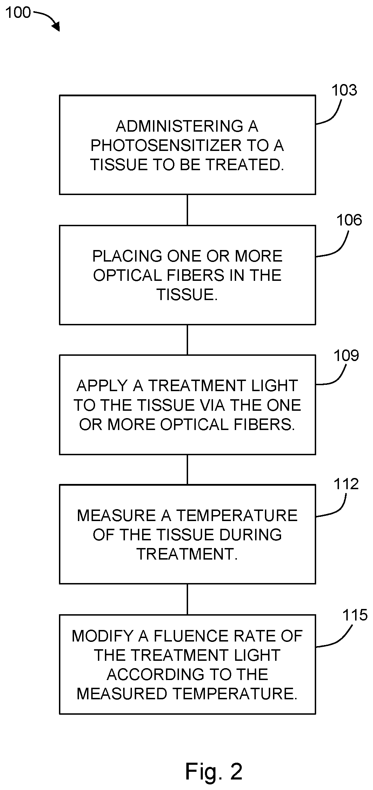

With reference to Figure 2, the present disclosure may be embodied as a method **100** for treating a tissue, for example, treating a tumor, of an individual using interstitial photodynamic therapy (I-PDT) (*see* Figure 2). The method **100** includes administering **103** a photosensitizer to the tissue. The photosensitizer may be, for example, porfimer sodium (Photofrin^{®}) or any other photosensitizer known for use in I-PDT-*e.g*., capable of generating reactive oxygen species and radicals when activated by light in the presence of oxygen. The photosensitizer may be administered **103** by, for example, intravenous injection.

One or more optical fibers are placed **106** into the tissue to be treated. The optical fibers may be placed at locations in the tissue according to a predetermined treatment plan. In some embodiments, the optical fiber(s) are placed **106** into the tissue by way of light-transmitting catheter(s) (LTCs) where each optical fiber is disposed in a light-transmitting catheter.

A treatment light is applied **109** to the tissue by way of the one or more optical fibers. The treatment light has a fluence (measured in, for example, joules per square centimeter-J/cm²) and a fluence rate (measured in, for example, milliwatts per square centimeter-mW/cm²).

The method **100** includes measuring **112** a temperature of the tissue during application of the treatment light (*i.e.,* during "treatment"). Measurement **112** of the tissue may be performed using any technique appropriate. For example, volumetric measurement may be accomplished using magnetic-resonance thermometry (MR thermometry or MRT). In another example, temperature is measured using a temperature-sensitive catheter. In embodiments where LTCs are placed in the tissue, a temperature-sensitive catheter may optionally be disposed in an LTC. Other methods for measuring **112** a temperature of a tissue may be used.

The fluence rate of the treatment light may be modified **115** based on the temperature of the tissue. The fluence rate of the treatment light within the tissue (*e.g*., intratumoral fluence rate) may be modified by adjusting the light dose rate. For example, the fluence rate may be decreased when the tissue temperature is higher than a (first) predetermined threshold. In this way, the temperature of the tissue will decrease. Similarly, the fluence rate of the treatment light may be increased if the tissue temperature is lower than a second predetermined threshold, which may be the same as or different from the first predetermined threshold. By increasing the fluence rate, the tissue temperature will increase. In exemplary embodiments, the fluence rate is modified to maintain a tissue temperature of between 50-65°C, inclusive. In another exemplary embodiment, the fluence rate is modified to maintain a tissue temperature of less than 60°C. In another exemplary embodiment, the fluence rate is modified to maintain a tissue temperature of substantially 60°C. By substantially, embodiments may maintain the temperature of the tissue within a desired tolerance, for example, ±5°C, ±1°C, ±0.5°C, ±0.2°C, or other tolerance levels between these exemplary values, for example, in 2°C increments. In embodiments incorporating thermal ablation, the temperature may be maintained at greater than 60°C and less than 100°C (or in some embodiments, less than 90°C) in order to avoid tissue carbonization.

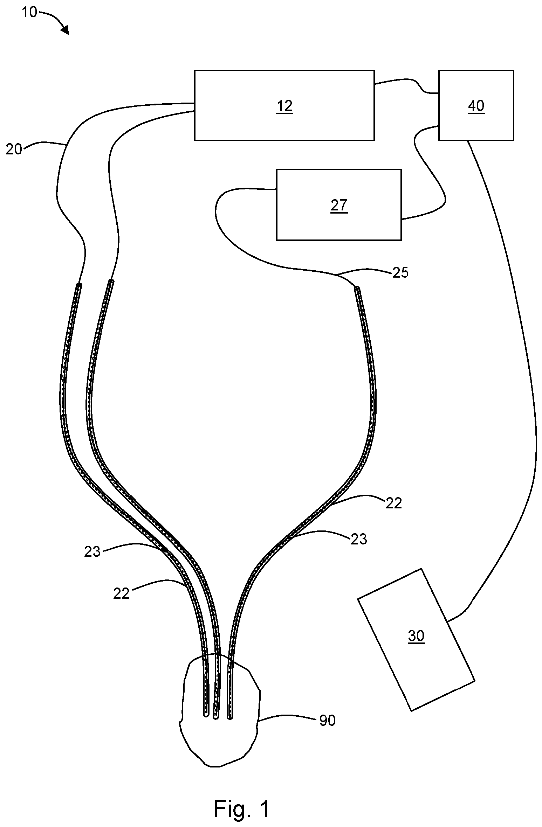

With reference to Figure 1, the present disclosure may be embodied as a system **10** for treating tissue **90** using I-PDT is provided. The system **10** includes a light source **12.** For example, the light source **12** may be a laser configured to emit light at a wavelength for activating a selected photosensitizer. For example, when using Photofrin, the emitted light may be 630 nm. An optical fiber **20** is operably coupled to the light source **12.** The optical fiber **20** is configured to deliver a light dose to the tissue **90.** The optical fiber **20** may have a cylindrical diffuser, a fiber with a flat-cut end, or other configuration. Embodiments of the system **10** may include additional optical fibers, for example, the embodiment depicted in Figure 1 includes a second optical fiber **20** for delivering a second light dose to the tissue **90.**

The system **10** includes a temperature sensor **30** for measuring a temperature of the tissue **90.** In some embodiments, the temperature sensor **30** may be configured to measure the temperature of the tissue **90** at more than one location, for example, more than one 3-D location within the tissue **90.** In some embodiments, the temperature sensor is a magnetic-resonance thermometer. In some embodiments, the temperature sensor comprises one or more temperature-sensitive catheters (*e.g*., a thermistor disposed in a catheter).

In some embodiments, the system **10** may include a light-transmitting catheter **22** (*e.g.,* a transparent catheter)(an "LTC"). In such embodiments, the optical fiber(s) **20** of the system **10** may be disposed within a corresponding number of LTCs **22.** For example, each optical fiber **20** may be disposed in a lumen **23** of an LTC **22.**

The system **10** further includes a controller **40** in communication with the temperature sensor **30.** The controller **40** is configured to modify a fluence rate of the treatment light based on the temperature of the tissue **90,** as described above. In some embodiments, the controller **40** is a programmable microprocessor, programmed to modify the treatment light based on a signal received from the temperature sensor. The treatment light may be modified by adjusting the intensity of the light source, attenuating the light emitted from the light source, independently attenuating the light in each optical fiber, or any other technique for increasing or decreasing the fluence rate of the treatment light delivered to the tissue via the optical fiber(s).

Some embodiments of the presently-disclosed system may include a dosimetry fiber **25** for measuring a light dose. In embodiments wherein the system includes an LTC **22,** the dosimetry fiber **25** may be disposed in an LTC **22,** for example, within a lumen **23** of an LTC **22.** The dosimetry fiber **25** may be operably coupled to a spectrometer **27** for measuring the light dose delivered to the tissue. In this way, a desired total dose may be delivered while also maintaining the temperature of the tissue in a desired range for effective photothermal ablation (or other synergistic effect when combined with I-PDT).

The present disclosure is further illustrated in the discussion below, which includes exemplary embodiments used to test the disclosed technique using mouse models.

### Further Discussion

The mechanism of action in photodynamic therapy (PDT) involves the generation of reactive oxygen species and radicals through light activation of photosensitizer in the presence of oxygen-*i.e*., an effective photoreaction. Previous studies demonstrated that oxygen-conserving light fluence rate (mW/cm²) is required for an effective photoreaction. In I-PDT, multiple optical fibers with a cylindrical diffuser or optical fibers with flat cut end are inserted into the tumor. During I-PDT, the light dose rate (mW/cm) dictates the resulted fluence rate in the tumor. Thus, the light fluence rate depends on the light dose rate delivered from the optical fibers.

In the U.S., Photofrin is the only approved photosensitizer in the treatment of obstructing esophageal, non-small cell endobronchial lung cancer and high-grade dysplasia in Barrett's esophagus. The FDA-approved light dose rate for I-PDT with Photofrin is 400 mW/cm length of the optical fiber cylindrical diffuser, which translates to a fluence rate of up to 800 mW/cm². This clinically-approved light dose rate induces significant thermal ablation that could overwhelm the photoreaction. However, our preclinical data suggest that this is not the case, and cure of locally advanced squamous cell carcinoma can be achieved in a mouse model treated with I-PDT and thermal ablation. In an ongoing preclinical study it was found that the laser light can induce significant heating that can induce immediate tissue ablation, at T > 60°C, without impeding the efficacy of the I-PDT.

The inventors hypothesized that the limited cure rate for I-PDT with porfimer sodium is due to the high dose rate that could limit the photodynamic efficiency by depleting tumor oxygen levels.

As further described below, aspects of the presently-disclosed method and system were implemented for testing. In particular, *in vivo* magnetic resonance thermometry (MRT) was used to quantify photothermal ablation during I-PDT. Time-to-event and cure rates were analyzed with Kaplan Meier curves. Safe and effective light dose rates and doses with photothermal ablation were identified.

### Animal Model

All procedures were carried out in accordance with a protocol approved by the Institutional Animal Care and Use Committee (IACUC) at Roswell Park Cancer Institute (RPCI). Female C3H, 8-12 weeks, mice bearing locally advanced SCCVII tumors were treated when the tumors reached a size of 9-10 mm in their largest diameter, and a volume of 400-500 mm³ calculated from caliper measurements, as is known in the art.

The animals were randomly assigned to receive light only (no drug) or I-PDT by administering 5 mg/kg porfimer sodium 24 hours prior to light delivery. Mice were considered cured if there was no palpable tumor any time at or after 60 days post I-PDT.

### Interstitial Photodynamic Therapy (I-PDT)

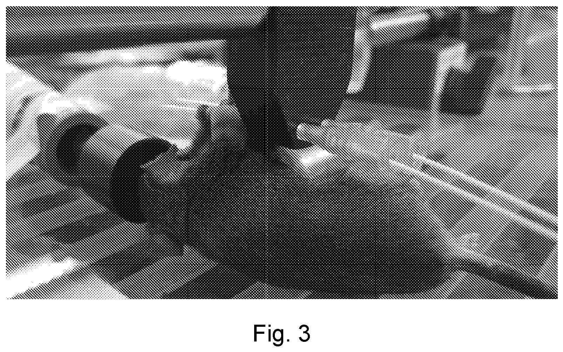

The laser light (treatment light) was delivered through one to three 0.98 mm diameter optical fibers with 20 mm cylindrical light diffuser RD20 (Medlight SA, Ecublens, Switzerland). The cylindrical light diffusers were connected to 1.0 Watt laser diode modules that emit 630±3 nm light (ML6500, Modulight Inc., Tampere, Finland). During light delivery, a custom made template was utilized to guide the placement of the laser fibers into the tumor, as shown in Figure 3.

### Magnetic Resonance Thermometry

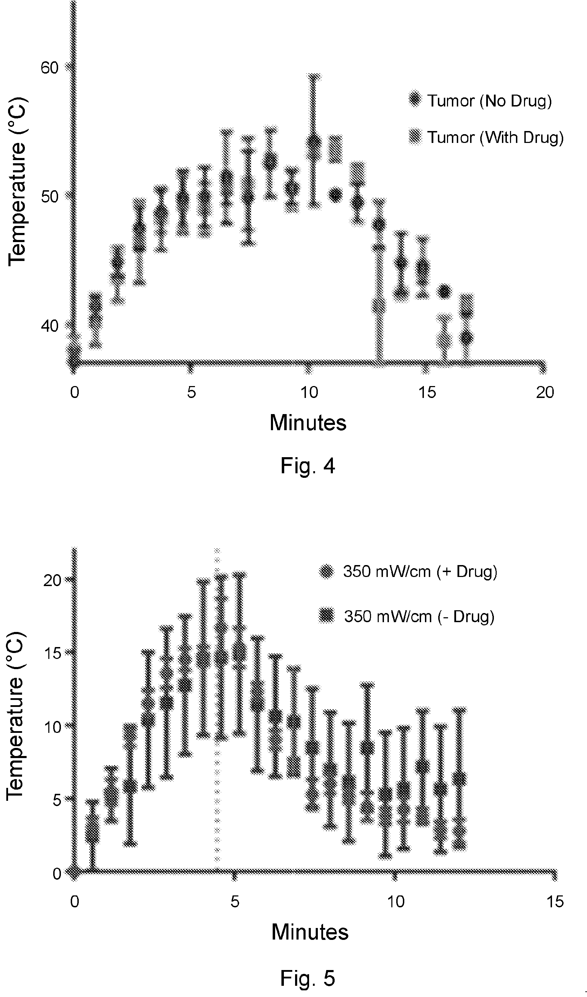

Magnetic resonance thermometry (MRT) by proton resonance frequency methods was carried out in a 4.7 Tesla preclinical scanner using the ParaVision 3.0.2 imaging platform (Bruker Biospin, Billerica MA) and a 35 mm quadrature transceiver coil. Figures 4 and 5 show that there was a temperature increase during the interstitial light delivery, with and without drug. These temperatures were averaged over the entire tumor volume.

### Results

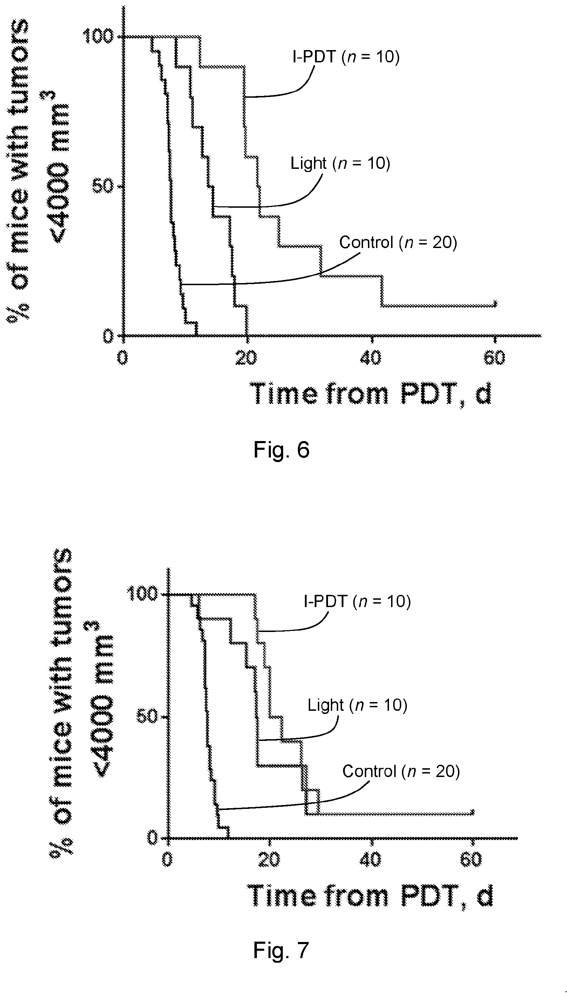

Figure 6 shows the percentage of mice with tumors less than 4000 mm³ over a period of 60 days post treatment. As shown in the chart, a control population was untreated, a second population was treated with a light dose of 150 mW/cm, 100 J/cm (control vs. light *p* ≤ 0.0001), and a third population was treated with I-PDT and similar dose (control vs. I-PDT *p* ≤ 0.0001; light vs. I-PDT *p* = 0.0004). Figure 7 is a similar chart for populations treated with a light dose of 350 mW/cm, 100 J/cm (control vs. light *p* ≤ 0.0001; control vs. I-PDT *p* ≤ 0.0001; light vs. I-PDT *p* = 0.339). It can be seen that after 60 days, only 10% of mice remained with tumors less than 4,000 mm³.

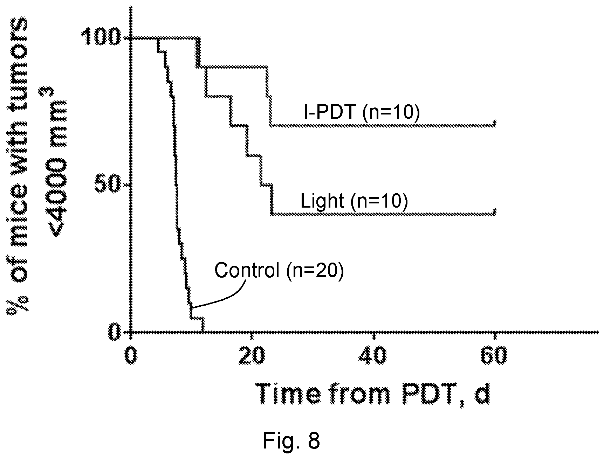

Figure 8 shows the beneficial results of an embodiment of the presently-disclosed techniques using concurrent I-PDT and thermal ablation. It can be seen that the use of light without photosensitizer results in a cure rate of 40%. Despite the previous consideration that a *lack* of significant temperature change of the tissue was a benefit of PDT, Figure 8 shows that concurrent photothermal ablation and I-PDT resulted in a cure rate of 70%.

Although claimed subject matter is described in terms of certain embodiments, other embodiments, including embodiments that do not provide all of the benefits and features set forth herein, are also within the scope of this disclosure. Various structural, logical, process step, and electronic changes may be made without departing from the scope of the disclosure.

## Claims

1. A system (10) for treating a tissue using interstitial photodynamic therapy (I-PDT), comprising:
a light source (12) configured to emit light at a wavelength for activating a photosensitizer;
an optical fiber (20) operably coupled to the light source and configured to deliver a light dose of treatment light to the tissue;
a temperature sensor (30) configured to measure a temperature of the tissue; and
a controller (40) in communication with the temperature sensor and configured to modify a fluence rate of the treatment light based on the measured temperature to maintain a tissue temperature between 60°C and 90°C.

2. The system of claim 1, wherein the temperature sensor is configured to measure temperature at a plurality of locations throughout the volume of the tissue.

3. The system of claim 2, wherein the temperature sensor is a magnetic resonance thermometer, or wherein the temperature sensor comprises a plurality of temperature sensitive catheters.

4. The system of any preceding claim, wherein the controller is configured to modify the fluence rate of the treatment light to maintain a tissue temperature of substantially 60°C.

5. The system of any preceding claim, further comprising a dosimetry fiber (25) for measuring the light dose.

6. The system of claim 5, further comprising a spectrometer operably coupled to the dosimetry fiber, or further comprising a light-transmitting catheter (22), wherein the optical fiber and/or the dosimetry fiber is disposed in the light-transmitting catheter.

7. The system of any preceding claim, further comprising:
a second optical fiber (20) operably coupled to the light source and configured to deliver a second light dose of treatment light to the tissue; and
wherein the controller is configured to modify a fluence rate of the second light dose based on the measured temperature.

8. The system of claim 7, wherein the fluence rate of the second light dose is modified based on a temperature at a second location of the tissue.

9. The system of any preceding claim, wherein the controller is a programmable microprocessor.

## Patentansprüche

1. System (10) zur Behandlung eines Gewebes unter Verwendung interstitieller photodynamischer Therapie (I-PDT), welches Folgendes umfasst:
eine Lichtquelle (12), die dazu konfiguriert ist, Licht mit einer Wellenlänge zum Aktivieren eines Fotosensibilisators zu emittieren;
eine optische Faser (20), die mit der Lichtquelle wirkgekoppelt ist und dazu konfiguriert ist, eine Lichtdosis von Behandlungslicht an das Gewebe zu liefern; einen Temperatursensor (30), der dazu konfiguriert ist, eine Temperatur des Gewebes zu messen; und
eine Steuerung (40), die in Kommunikation mit dem Temperatursensor steht und dazu konfiguriert ist, eine Fluenzrate des Behandlungslichts basierend auf der gemessenen Temperatur zu modifizieren, um eine Gewebetemperatur zwischen 60 °C und 90 °C aufrechtzuerhalten

2. System nach Anspruch 1, wobei der Temperatursensor dazu konfiguriert ist, eine Temperatur an mehreren Stellen über das gesamte Volumen des Gewebes zu messen.

3. System nach Anspruch 2, wobei der Temperatursensor ein Magnetresonanzthermometer ist oder wobei der Temperatursensor mehrere temperaturempfindliche Katheter umfasst.

4. System nach einem vorhergehenden Anspruch, wobei die Steuerung dazu konfiguriert ist, die Fluenzrate des Behandlungslichts zu modifizieren, um eine Gewebetemperatur von im Wesentlichen 60 °C aufrechtzuerhalten

5. System nach einem der vorhergehenden Ansprüche, das ferner eine Dosimetriefaser (25) zum Messen der Lichtdosis umfasst.

6. System nach Anspruch 5, das ferner ein Spektrometer umfasst, das mit der Dosimetriefaser wirkgekoppelt ist, oder das ferner einen lichttransmittierenden Katheter (22) umfasst, wobei die optische Faser und/oder die Dosimetriefaser in dem lichttransmittierenden Katheter angeordnet ist.

7. System nach einem vorhergehenden Anspruch, das ferner Folgendes umfasst:
eine zweite optische Faser (20), die mit der Lichtquelle wirkgekoppelt ist und dazu konfiguriert ist, eine zweite Lichtdosis von Behandlungslicht an das Gewebe liefern; und
wobei die Steuerung dazu konfiguriert ist, eine Fluenzrate der zweiten Lichtdosis basierend auf der gemessenen Temperatur zu modifizieren.

8. System nach Anspruch 7, wobei die Fluenzrate der zweiten Lichtdosis basierend auf einer Temperatur an einer zweiten Stelle des Gewebes modifiziert wird.

9. System nach einem vorhergehenden Anspruch, wobei die Steuerung ein programmierbarer Mikroprozessor ist.

## Revendications

1. Système (10) de traitement d'un tissu par thérapie photodynamique interstitielle (I-PDT), comprenant :
une source de lumière (12) configurée pour émettre une lumière à une longueur d'onde pour activer un photosensibilisateur ;
une fibre optique (20) couplée fonctionnellement à la source de lumière et configurée pour délivrer une dose de lumière de traitement au tissu ;
un capteur de température (30) configuré pour mesurer une température du tissu ; et
un dispositif de commande (40) en communication avec le capteur de température et configuré pour modifier un débit de fluence de la lumière de traitement sur la base de la température mesurée afin de maintenir une température tissulaire entre 60 °C et 90 °C.

2. Système selon la revendication 1, le capteur de température étant configuré pour mesurer la température en une pluralité d'emplacements dans tout le volume du tissu.

3. Système selon la revendication 2, le capteur de température étant un thermomètre à résonance magnétique, ou le capteur de température comprenant une pluralité de cathéters sensibles à la température.

4. Système selon l'une quelconque des revendications précédentes, le dispositif de commande étant configuré pour modifier le débit de fluence de la lumière de traitement afin de maintenir une température tissulaire sensiblement de 60 °C.

5. Système selon l'une quelconque des revendications précédentes, comprenant en outre une fibre dosimétrique (25) pour mesurer la dose de lumière.

6. Système selon la revendication 5, comprenant en outre un spectromètre couplé de manière opérationnelle à la fibre dosimétrique, ou comprenant en outre un cathéter transmettant la lumière (22), la fibre optique et/ou la fibre dosimétrique étant disposée dans le cathéter transmettant la lumière.

7. Système selon n'importe quelle revendication précédente, comprenant en outre :
une seconde fibre optique (20) couplée fonctionnellement à la source de lumière et configurée pour délivrer une seconde dose de lumière de lumière de traitement au tissu ; et
le dispositif de commande étant configuré pour modifier un débit de fluence de la seconde dose de lumière sur la base de la température mesurée.

8. Système selon la revendication 7, le débit de fluence de la seconde dose de lumière étant modifié sur la base d'une température à un second emplacement du tissu.

9. Système selon l'une quelconque des revendications précédentes, le dispositif de commande étant un microprocesseur programmable.
